# EUROPEAN PATENT APPLICATION

(11) **EP 0 729 031 A1**
(43) Date of publication of application: **28.08.1996**
(21) Application number: 96102222.5
(22) Date of filing: 15.02.1996
(51) Int. Cl.: G01N 33/72

(54) **Set of reagents determining the content of total haemoglobin**

(30) Priority: 24.02.1995 EP 95102635
(71) Applicant: F. HOFFMANN-LA ROCHE AG, 4002 Basel (CH)
(72) Inventor: Bona, Vittorio, CH-4133 Pratteln (CH); Vorberg, Ewald, D-79104 Freiburg (DE); Witzigmann, Achim, D-79276 Reute (DE)
(74) Representative: Cottong, Norbert A.

(57) **Abstract**

The invention relates to reagents and methods for determining the content of total haemoglobin in a blood sample or a sample derived from blood.

The invention proposes:
- a set of reagents for determining the content of total haemoglobin in a blood sample or a sample derived from blood, comprising a haemolysis reagent which is an acidic solution having a pH between 0.5 and 5.0, preferably between 0.5 to 3.0, and a green chromophore forming reagent which is a basic solution having a pH between 7.0 and 12.0, preferably between 9.0 and 11.5, and containing a non-ionic detergent and/or an anionic detergent.
- a method of determining the content of total haemoglobin in a blood sample or a sample derived from blood which uses the above set of reagents.

## Description

The invention relates to a set of reagents for determining the content of total haemoglobin in a blood sample or a sample derived from blood, said set comprising a haemolysis reagent which is an acidic solution having a pH between about 0.5 and 5.0, preferably between about 0.5 and 3.0, and a green chromophore forming reagent, which is a basic solution having a pH between about 7.0 and 12.0, preferably between about 9.0 and 11.5, and containing a non-ionic detergent and/or an anionic detergent. The invention further relates to a set of reagents for determining both the content of total haemoglobin and the content of a particular haemoglobin derivative in a blood sample or a sample derived from blood.

The invention also concerns a method for determining the content of total haemoglobin, and preferably also the content of a particular haemoglobin derivative, in a blood sample or a sample derived from blood, and the use of the above set of reagents in such a method.

The expression "a green chromophore forming reagent" means a reagent capable of forming a greenish product, hereafter referred to as green chromophore, with the haemolysate obtained after treating a blood sample or a sample derived from blood with the above defined haemolysis reagent, whereby the green chromophore has an absorption spectrum similar to that of alkaline haematin D-575 described in British Patent Publication No. 2 052 056. The green chromophore is the stable reaction product of all forms of haemoglobin with the green chromophore forming reagent.

British Patent Publication No. 2 052 056 describes a simple method for spectrometric determination of the total haemoglobin content in blood, consisting of adding to the blood sample a reagent containing a water-soluble non-ionic detergent in a strongly alkaline solution of pH between 12.7 and 13.7, incubating for at least one minute and measuring the absorption at a wave length of about 575 nm. This method is based on the fact that all haemoglobin derivatives under those conditions are converted into a uniform stable reaction product, called alkaline hematin D-575, a green chromophore having a maximum of absorption at 575 nm. This method however has the drawback that the strongly alkaline reagent tends to absorb ambient carbon dioxide and thus become less basic, thereby limiting preservation of this reagent under conditions of use, especially in automatic clinical chemistry analyzers.

European Patent Publication No. 559 164 discloses another method for determining the total haemoglobin content in a blood sample, consisting of adding to the blood sample a reagent containing a ionic detergent in a substantially neutral solution of pH between 5.0 and 9.5, incubating for a period of 1 to 10 minutes and measuring the absorption at a wavelength of 540 nm. The reagent lyses the erythrocytes contained in the blood sample and reacts with the haemoglobin released so as to convert it quantitatively into a green chromophore. This method has the drawback that it shows a strong interference with lipids and therefore gives false results for patients with an elevated level of lipids, as is often the case for diabetic patients.

Japanese Patent Publication No. 227171/1985 describes another method of determining the total content of haemoglobin consisting of washing a sample of whole blood with a physiological saline solution to obtain the erythrocytes, lysing the erythrocytes in a neutral solution and treating the sample obtained with a solution of pH of at least 11.5 containing TRITON® X100, so as to convert all haemoglobin forms into a green chromophore. This method also has the drawback that the alkaline reagent tends to absorb ambient carbon dioxide and thus become less basic. The reaction used for determination of the total content of haemoglobin is very sensitive to a variation of pH of the alkaline reagent.

The problem addressed by the present invention is to find a set of reagents and a method for spectrophotometric determination of the content of total haemoglobin, as well as for spectrophotometric determination of both the content of total haemoglobin and the content of a particular haemoglobin derivative, in a blood sample or a sample derived from blood, that does not have the above drawbacks.

This problem is solved by the invention as defined in the set of appended claims.

The set of reagents of the invention and the method using same have the surprising advantages that the determination of total haemoglobin is not affected by a variation of pH of the green chromophore forming reagent, nor by the presence of lipids in the blood sample or the sample derived from blood.

The haemolysis reagent preferably contains a protease which is active in a pH range comprised between about 0.5 and 5.0.

An example of such a protease is pepsin. This protease is effective when used at a concentration of at least 10 kU/ml, preferably at least 100 kU/ml.

For preserving the activity of the protease, particularly when the protease is pepsin, it is interesting to add a small amount, preferably corresponding to a concentration of at least 0.01 % by weight, of a non-ionic detergent such as isotridecyl poly(ethyleneglycol)₈ (GENAPOL® X80 - Boehringer Mannheim) or a sorbitanester (e.g. TWEEN® - ICI). Such non ionic detergents have the further advantage of stabilizing the haemolysate obtained.

The haemolysis reagent advantageously contains a small amount of an agent capable of oxidizing haem. An example of such an agent is 5-bromo-5-nitro-1,3-dioxane, which is preferably used at a concentration of about 0.01 to 0.1% (w/v). That agent, which can for instance conveniently be found as the main constituent of BRONIDOX K (Henkel) or at a concentration of about 10% in propyleneglycol in BRONIDOX L (Henkel), also prevents microbial growth.

The detergent contained in the green chromophore forming reagent may be an anionic detergent or a non-ionic detergent. This detergent apparently helps in unfolding the protein, so as to make the haem group more accessible and oxidizable.

Suitable non ionic detergents are for instance isotridecyl poly(ethyleneglycol)₈ (GENAPOL® X80 - Boehringer Mannheim), 4-(1,1,3,3-tetramethylbutyl)-phenol ethoxylate (TRITON® X-100 - Rohm & Haas), polyethyleneglycol-lauryl-ether, polyethyleneglycolcetyl-ether, polyethyleneglycol-oleyl-ether, polyethyleneglycol-stearyl-ether, or a mixture of such ethers (e.g. BRIJ® 58 - Atlas Chemie). 4-(1,1,3,3-tetramethylbutyl)-phenol ethoxylate and isotridecyl-poly(ethyleneglycol)₈ are particularly suitable, the latter detergent being preferred. The non-ionic detergent is generally used at a concentration between about 5 and 100 mM, preferably between about 10 and 70 mM.

A clarifying agent such as a cholate salt, e.g. sodium cholate, is preferably added in order to avoid turbidity problems often encountered with non-ionic detergents at a temperature higher than room temperature, e.g. about 37 C°, which is generally-used in automatic analyzers. The concentration of the clarifying agent in the green chromophore forming reagent is preferably from about 6 to about 18 mM.

Suitable anionic detergents are for instance sodium dodecylsulfate (SDS), sodium dioctylsulfosuccinate (DONS) or SULFORIZINATE (made from olive or ricinus oil with sulfuric acid, available from Chemische Fabrik G. Zimmerli A.G., CH-4663 Aarburg, Switzerland).

The invention also relates to a set of reagents for determining both the content of total haemoglobin and the content of a particular haemoglobin derivative in a blood sample or a sample derived from blood, said set comprising
- a haemolysis reagent as described above,
- a green chromophore forming reagent as described above, and
- a reagent for determining the content of a particular haemoglobin derivative.

The reagent for determining the content of a particular haemoglobin derivative is a reagent that allows determining, preferably without any prior purification step, the amount of a particular haemoglobin derivative in the haemolysate resulting from treatment of the sample with the above haemolysis reagent. An interesting example of such a reagent is an immunoreagent comprising an antibody, preferably a monoclonal antibody, that specifically binds to a particular haemoglobin derivative.

Haemoglobin derivatives for which such a set of reagents is useful are all haemoglobin forms present in blood which are of clinical interest, particularly glycated haemoglobin derivatives, such as haemoglobin A1a, haemoglobin A1b and haemoglobin A1c. The above set of reagents allows to determine the ratio between the content of a particular glycated haemoglobin derivative and the content of total haemoglobin. That ratio reflects the average glucose level in blood over a period of time and hence represents an interesting parameter for monitoring metabolic control in diabetes.

The above set of reagents is particularly adapted to the determination of the ratio between the content of haemoglobin A1c and the content of total haemoglobin. In such a case, the reagent for determining the content of haemoglobin A1c is preferably an immunoreagent comprising at least one monoclonal antibody which specifically recognizes this protein. An example of such a monoclonal antibody is the monoclonal antibody disclosed in US Patent No. 4,727,036.

The invention also relates to a method for determining the content of total haemoglobin in a blood sample or a sample derived from blood, said method comprising the steps of
(a) treating the sample with the above described haemolysis reagent, and
(b) incubating the resulting haemolysate with the above described green chromophore forming reagent, for a sufficient time so as to convert all haemoglobin derivatives into a green chromophore, and measuring the absorbance of the solution obtained.

The haemolysis step (a) is preferably performed by mixing the sample with the haemolysis reagent for a period of at least about 5 seconds, preferably from about 5 seconds to about 5 minutes.

The reaction time to convert all haemoglobin derivatives of the haemolysate into the green chromophore is generally not more than about 250 seconds, preferably not more than about 100 seconds.

The absorbance of the solution is measured at a wave length where the green chromophore has an extinction coefficient sufficiently higher than that of the other components present in the sample, generally between about 500 and 600 nm, particularly between about 540 and 590 nm. A particularly advantageous wave length is about 570 nm.

An important advantage of the method of the invention is that the reaction of conversion of all haemoglobin derivatives into a green chromophore, shows almost no sensitivity to a variation of pH of the green chromophore forming reagent.

When the absorbance of the green chromophore is measured at a wave length of about 550 nm, a variation of the pH of the green chromophore forming reagent from about 11.5 to about 9.0, e.g. due to absorption of carbon dioxide, does not significantly affect the results in the determination of total haemoglobin.

When the absorbance of the green chromophore is measured at a wave length of about 570 nm, a variation of the pH of the green chromophore forming reagent from about 11.5 to about 7.0, does not significantly affect the results in the determination of total haemoglobin.

The method of the invention has the advantage, in contrast to the method described in European Patent Publication No. 559 164, that the presence of lipids at physiological concentrations does not interfere with the determination of haemoglobin.

The invention also concerns a method for determining both the content of total haemoglobin and the content of a particular haemoglobin derivative in a blood sample or a sample derived from blood, said method comprising the steps of
(a) treating the sample with the above described haemolysis reagent,
(b) incubating an aliquot of the resulting haemolysate with the above described green chromophore forming reagent, for a sufficient time so as to convert all haemoglobin derivatives into a green chromophore, and measuring the absorbance of the solution obtained, and
(c) determining the content of the particular haemoglobin derivative in another aliquot of the haemolysate.

The above method is particularly adapted to the determination of the ratio between the content of haemoglobin A1c and the content of total haemoglobin.

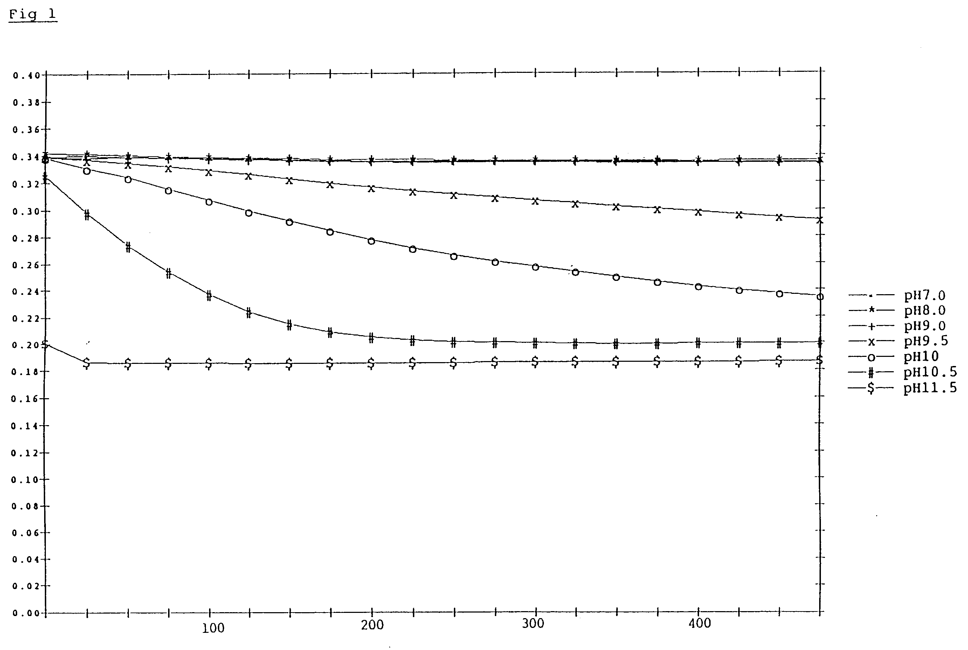

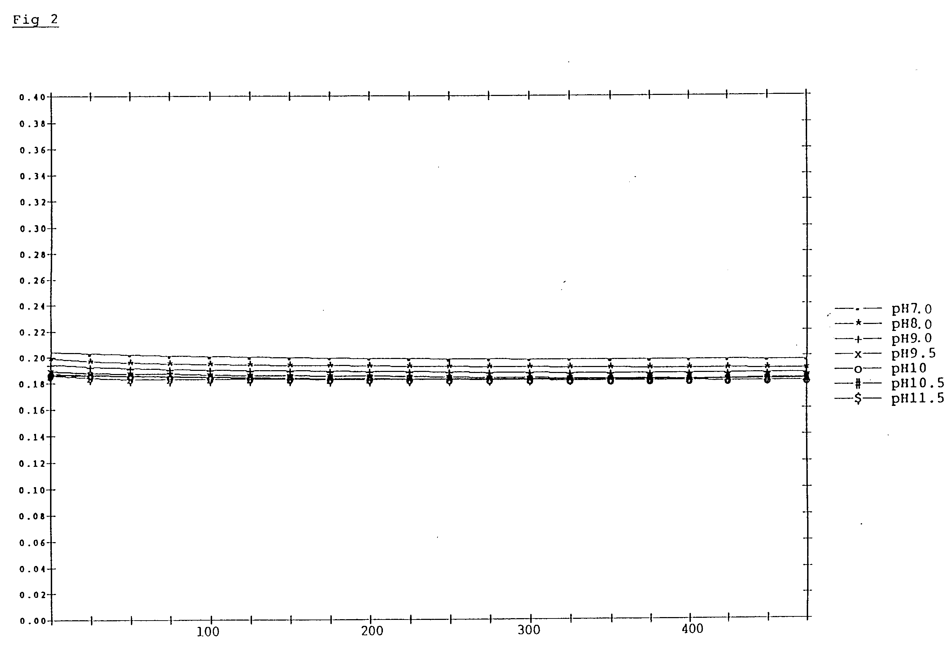

The present invention will be further illustrated by the following examples. The following description will be better understood by referring to Figures 1 and 2.

Figure 1 represents the variation as a function of time of the absorbance at a wave length of about 550 nm, of the reaction product between different green chromophore forming reagents having a pH from 11.5 to 7.0 and the haemolysate obtained by treating the blood sample with water.

Figure 2 represents the variation as a function of time of the absorbance at a wave length of about 550 nm of the reaction product between different green chromophore forming reagents having a pH from 11.5 to 7.0 and the haemolysate obtained by treating the blood sample with an haemolysis reagent containing 0.02% 5-bromo-5-nitro-1,3-dioxane (BRONIDOX K-Henkel), 200 kU/l porcine pepsin, 20 mM citrate buffer pH 2.4.

The curves corresponding to the green chromophore forming reagents having a pH from 11.5 to 7.0 represent how the green chromophore forming reaction, which is the basis of the spectrophotometric determination of the total content of haemoglobin, is affected by a change of pH of the green chromophore forming reagent.

### Example 1:

Sensitivity of the total haemoglobin determination reaction to a variation of pH of the green chromophore forming reagent for a haemolysis step performed in a neutral solution.

### 1) Preparation of green chromophore forming reagents

The following green chromophore forming reagents were prepared:
- a green chromophore forming reagent containing 40 mM GENAPOL® X-80 (Boehringer Mannheim) , 12 mM sodium cholate, 400 mM potassium phosphate buffer of pH 11.5,
- a green chromophore forming reagent containing 40 mM GENAPOL® X-80, 12 mM sodium cholate, 400 mM potassium carbonate of pH 10.5, 10.0 or 9.5,
- a green chromophore forming reagent containing 40 mM GENAPOL® X-80, 12 mM sodium cholate, 400 mM Tris/HCl of pH 9.0 or 8.0,
- and a green chromophore forming reagent containing 40 mM GENAPOL® X-80, 12 mM sodium cholate, 400 mM potassium phosphate pH 7.0.

### 2) Treating of the blood sample with water and reacting the haemolysate with the green chromophore forming reagents.

This reaction consisted in mixing the blood sample with demineralized water for about 1 minute, then mixing the haemolysate obtained with the above described green chromophore forming reagents and spectrometrically following at a wave length of about 550 nm the formation of the green chromophore.

Figure 1 represents the variation as a function of time of the absorbance at about 550 nm for the above described green chromophore forming reagents having a pH between 11.5 and 7.0.

Figure 1 shows that
- for a pH of 11.5 of the green chromophore forming reagent, the green chromophore forming reaction is completed after about 100 seconds,
- for a pH of 10.5 of the green chromophore forming reagent, the reaction is completed after about 250 seconds,
- for a pH of 10.0 of the green chromophore forming reagent, the reaction is not completed after about 450 seconds, and
- for a pH of 9.5 and below of the green chromophore forming reagent, the reaction proceeds very slowly or does not take place.

A short time for completion of the chromophore forming reaction is desirable for a speedy assay for determining total haemoglobin. When using a clinical chemistry analyzer the instrument is set so as to measure the absorbance value after a given reaction time, e.g. about 100 seconds. The absorbance value is proportional to the amount of total haemoglobin in the sample only if the quantitative reaction of conversion of all haemoglobin derivatives into the green chromophore has been completed. The above results thus indicate that a variation of the pH of the green chromophore forming reagent, e.g. due to absorption of carbon dioxide, will lead to errors in the determination of total haemoglobin in a blood or sample derived from blood.

### Example 2:

Influence of the haemolysis step on the sensitivity of the total haemoglobin determination reaction to a variation of pH of the green chromophore forming reagent.

### 1) Methodology

### Preparation of different haemolysis reagents

Numerous different haemolysis reagents of pH between 0.5 and 5.0, containing or not containing a protease active in this range of pH and/or an agent capable of oxidizing haem were prepared. We set out hereafter examples of prepared such haemolysis reagents:
- haemolysis reagent containing 20 mM citrate buffer of pH 5.0, 4.0, 3.0, or 2.4,
- haemolysis reagent containing hydrochloric acid of pH 2.0, 1.5, 1.0 or 0.5
- haemolysis reagent containing 20 mM citrate buffer of pH 2.4, and 200 kU/l porcine pepsin, 100 kU/l porcine pepsin, 50 kU/l porcine pepsin, 25 kU/l porcine pepsin, or 10 kU/l porcine pepsin.
- haemolysis reagent containing 0.02% 5-bromo-5-nitro-1,3-dioxane (BRONIDOX K- Henkel),
- haemolysis reagent containing 0.02% 5-bromo-5-nitro-1,3-dioxane (BRONIDOX K-Henkel), 20 mM citrate buffer pH 2.4,
- and haemolysis reagent containing 0.02% 5-bromo-5-nitro-1,3-dioxane (BRONIDOX K- Henkel), 200 kU/l porcine pepsin, 20 mM citrate buffer pH 2.4.

The influence of the haemolysis step on the sensitivity of the total haemoglobin determination reaction to a variation of pH of the green chromophore forming reagent was studied by mixing the blood sample with different haemolysis reagents for about 1 minute and mixing the haemolysate obtained with the green chromophore forming reagents prepared in Example 1 1) and another green chromophore forming reagent having a pH of 12.0 (containing 40 mM GENAPOL® X-80, 12 mM sodium cholate, and 400 mM potassium phosphate buffer), and spectrometrically following at a wave length of about 550 nm the formation of the green chromophore.

The absorbance spectrum was determined for the green chromophore obtained.

### 2) Results

### a) Influence of the pH of the haemolysis reagent

Decreasing the pH of the haemolysis reagent increases the reactivity of the haemolysate with the green chromophore forming reagent (and thus reduces sensitivity of the total haemoglobin determination reaction to the pH of the green chromophore forming reagent), this effect being significant for a pH of 5.0 and below. Lowering the pH of the green chromophore forming reagent increases the absorbance at a wave length of about 550 nm after completion of the green chromophore forming reaction, hereafter referred to as the 550 end-point absorbance. This increase of the 550 end-point absorbance is due to a slight change of the absorbance spectrum of the green chromophore. All determined spectra of the green chromophore have the same absorbance at a wave length of about 570 nm.

For instance, for the above described haemolysis reagent containing 20 mM citrate buffer pH 2.4, the 550 end-point absorbance is reached after a maximum of about 250 seconds for all green chromophore forming reagents having a pH from 12.0 to 7.0, the change in the 550 end-point absorbance being from about 0.185 for a pH of the green chromophore forming reagent of 11.5, to about 0.245 for a pH of the green chromophore forming reagent of 7.0. Those results compared to those obtained in Example 1 under paragraph 2) illustrate the reactivity increase and the change of the 550 end-point absorbance mentioned in the preceding paragraph.

### b) Influence of the presence of pepsin

Addition of pepsin to a haemolysis reagent having a pH where this protein is sufficiently active, i.e. approximately up to a pH of about 3.0, increases the reactivity of the haemolysate with the green chromophore forming reagent. This increase in reactivity is significant for a concentration of pepsin of at least 10kU/l. As under a) lowering the pH of the green chromophore forming reagent increases the 550 end-point absorbance but to a lesser extent than when no protease is present. All determined spectra of the green chromophore have the same absorbance at a wave length of about 570 nm.

For instance, for the above described haemolysis reagent containing 200 kU/l porcine pepsin, 20 mM citrate buffer pH 2.4, the 550 end-point absorbance is reached after a maximum of about 100 seconds for all green chromophore forming reagents having a pH from 12.0 to 7.0, the change in the 550 end-point absorbance being from about 0.185 for a pH of the green chromophore reagent of 12.0, to about 0.22 for a pH of the green chromophore forming reagent of 7.0. Those results compared to those obtained under paragraph a) above illustrate the reactivity increase and the change to a lesser extent of the 550 end-point absorbance mentioned in the preceding paragraph.

### c) Influence of the presence of an agent capable of oxidising haem

Addition of 5-bromo-5-nitro-1,3-dioxane slightly increases the reactivity of the haemolysate with the green chromophore forming reagent. As under a) and b) lowering the pH of the green chromophore forming reagent increases the 550 end-point absorbance but to a lesser extent than when no agent capable of oxidizing haem is present. All determined spectra of the green chromophore have the same absorbance at a wave length of about 570 nm.

For instance, for the above described haemolysis reagent containing 0.2% (w/v) 5-bromo-5-nitro-1,3-dioxane, 200 kU/l porcine pepsin, 20 mM citrate buffer pH 2.4, the 550 end-point absorbance is reached after a maximum of 100 seconds for all green chromophore forming reagents having a pH from 12.0 to 7.0, the change in the 550 end-point absorbance being from about 0.18 for a pH of the green chromophore reagent of 12, to about 0.195 for a pH of the green chromophore forming reagent of 7.0. Those results compared to those obtained under paragraph b) above illustrate the reactivity increase and the change to a lesser extent of the 550 end-point absorbance mentioned in the preceding paragraph.

The results mentioned in the preceding paragraph can be seen on Figure 2, which represents the variation as a function of time of the absorbance at a wave length of 550 nm of the reaction product between different green chromophore forming reagents having a pH from 11.5 to 7.0 and the haemolysate obtained by treating the blood sample with the above haemolysis reagent.

Figure 2 shows that a decrease of the pH of the green chromophore forming reagent from 11.5 to 9.0, e.g. due to carbon dioxide absorption, will not significantly affect the time at which the 550 end-point absorbance is reached, nor the value of the latter, and hence will not lead to errors in the measurement of total haemoglobin content when the absorbance is measured at about 550 nm.

Since the green chromophore obtained has the same end-point absorbance at a wave length of about 570 nm for green chromophore forming reagents having a pH from 12.0 to 7.0, a change of pH within this range does not lead to errors in the measurement of total haemoglobin content if the absorbance is measured at this wave length.

### Example 3:

Study of interference of lipids on the determination of total haemoglobin for the method of the invention.

The influence of possible lipids in blood samples was studied by adding increasing concentrations up to 2 g/l of INTRALIPID (containing per ml 200 mg Soyae ol. fract., 12 mg fract. e vitello ovi and 22.5 mg glycerol, available from Kabi Vitrum - Stockholm, Sweden) to blood samples containing 200 mg/ml of total haemoglobin and measuring the total haemoglobin content with a set of reagents according to the invention (comprising a haemolysis reagent of pH of about 2.4 containing 300 kU/l porcine pepsin, 20 mM citric acid, 0.02 % (w/v) 5-bromo-5-nitro-1,3-dioxane, and a green chromophore forming reagent containing 40 mM GENAPOL X-80, 12 mM sodium cholate, 400 mM potassium phosphate buffer pH 11.5.

No interference was found using the set of reagents of the invention.

## Claims

1. A set of reagents for determining the content of total haemoglobin in a blood sample or a sample derived from blood, said set comprising
- a haemolysis reagent which is an acidic solution having a pH between about 0.5 and 5.0, preferably between about 0.5 to 3.0, and
- a green chromophore forming reagent which is a basic solution having a pH between about 7.0 and 12.0, preferably between about 9.0 and 11.5, and containing a non-ionic detergent and/or an anionic detergent.

2. A set according to claim 1, wherein the haemolysis reagent contains a protease.

3. A set according to claim 2, wherein the haemolysis reagent contains pepsin as a protease.

4. A set according to claim 3, wherein the haemolysis reagent contains pepsin at a concentration of at least 10 kU/l, preferably at least 100 kU/l.

5. A set according to any of claims 2 to 4, wherein the haemolysis reagent further contains a non-ionic detergent.

6. A set according to any of claims 1 to 5, wherein the haemolysis reagent contains an agent capable of oxidizing haem.

7. A set according to claim 6, wherein the agent capable of oxidizing haem is 5-bromo-5-nitro-1,3-dioxane.

8. A set according to any of claims 1 to 7, wherein the chromophore forming reagent contains isotridecyl poly(ethyleneglycol)₈ as a detergent.

9. A set of reagents for determining both the content of total haemoglobin and the content of a particular haemoglobin derivative in a blood sample or a sample derived from blood, said set comprising
- a haemolysis reagent as defined in any of claims 1 to 7,
- a green chromophore forming reagent as defined in any of claims 1 and 8, and
- a reagent for determining the content of a particular haemoglobin derivative.

10. A set of reagents according to claim 9 wherein the haemoglobin derivative to be determined is haemoglobin A1c.

11. A method for determining the content of total haemoglobin in a blood sample or a sample derived from blood, said method comprising the steps of
(a) treating the sample with a haemolysis reagent as defined in any of claims 1 to 7, and
(b) incubating the resulting haemolysate with an green chromophore forming reagent as defined in any of claims 1 and 8, for a sufficient time so as to convert all haemoglobin derivatives into a green chromophore, and measuring the absorbance of the solution obtained.

12. A method for determining both the content of total haemoglobin and the content of a particular haemoglobin derivative in a blood sample or a sample derived from blood, said method comprising the steps of
(a) treating the sample with a haemolysis reagent as defined in any of claims 1 to 7,
(b) incubating an aliquot of the resulting haemolysate with a green chromophore forming reagent as defined in any of claims 1 and 8, for a sufficient time so as to convert all haemoglobin derivatives into a green chromophore, and measuring the absorbance of the solution obtained, and
(c) determining the content of the particular haemoglobin derivative in another aliquot of the haemolysate.

13. A method according to claim 12 wherein the haemoglobin derivative is haemoglobin A1c.

14. Use of a set of reagents according to any of claims 1 to 8 for determining the content of total haemoglobin in a blood sample or a sample derived from blood.

15. Use of a set of reagents according to claim 9 for determining the ratio between the content of a particular haemoglobin derivative and the content of total haemoglobin in a blood sample or a sample derived from blood.

16. Use of a set of reagents according to claim 10 for determining the ratio between the content of haemoglobin A1c and the content of total haemoglobin in a blood sample or a sample derived from blood.
